# EUROPEAN PATENT APPLICATION

(11) **EP 2 189 520 A1**
(43) Date of publication of application: **26.05.2010**
(21) Application number: 07742967.8
(22) Date of filing: 08.05.2007
(51) Int. Cl.: C12N 1/02, C12N 5/04, C12N 5/06

(54) **CELL ISOLATION METHOD**

(71) Applicant: Yanagita, Tomotaka, Tokyo 156-0052 (JP); Yamashita, Koji, Toshima-ku Tokyo 171-0041 (JP); Kageyama, Kotaro, 55-3 Sakuradai 2-chome Nerima-ku Tokyo 176-0002 (JP); Tsuji, Takashi, 20-2, Nanakuni 5-chome Hachiouji-shi Tokyo 192-0919 (JP)
(72) Inventor: TSUJI, Takashi, Tokyo 192-0919 (JP); WADA, Hidenori, Tokyo 154-0022 (JP)
(74) Representative: Giovannini, Francesca
(86) International application number: PCT/JP2007/059532
(87) International publication number: WO 2008/139572

(57) **Abstract**

The present invention is a method for isolating a desired cell, which comprises selectively applying culture conditions including a culture medium to a sample potentially containing various cells, with addition of a cell enlarger simultaneously with, before or after the application. Thereby, it is possible to conveniently and efficiently obtain unknown but useful microorganisms occurring in a natural environment that are less competitive.

## Description

### TECHNICAL FIELD

The present invention is a technique related to methods for conveniently and efficiently isolating and obtaining a great variety of unknown but useful microorganisms occurring in a natural environment. In particular, the present invention is a technique related to methods intended for obtaining or knowing distribution of unknown but useful microorganisms occurring in a natural environment that are less competitive and difficult to find.

### BACKGROUND ART

In a natural environment, a great variety of microorganisms occur, many of which produce substances useful for human being or decompose harmful substances to provide great benefits.

For instance, among such microorganisms are soil microorganisms such as denitrificans. In recent years, environmental problems such as water system contamination by nitrate nitrogen and generation of N₂O (nitrous oxide) as a greenhouse gas are serious due to a volume use of nitrogenous fertilizers, an increase of imported foodstuff and feedstuff and the like. For solution of such problems, denitrificans are expected to play a huge role. Specifically, in rice paddy soil, ammonia (NH₄⁺) derived from nitrogenous fertilizers and domestic wastewater is present and part of it is absorbed by rice plant while the rest of it is first converted by the action of nitrifying bacteria to nitrate ion (NO₃⁻) and finally converted by denitrificans to harmless nitrogen gas (N₂) instead of nitrogen oxides to be returned to the atmosphere. Therefore, rice paddy soil is an excellent farmland with less leaching of nitric acid and generation of nitrogen oxides such as N₂O in comparison with field soil or grassland soil by virtue of the cooperative action of nitrifying bacteria and denitrificans. At present, however, although various microorganisms that exhibit denitrifying capabilities (denitrificans) under artificial culture conditions are known, reliable identification results concerning the species of denitrificans actually active in rice paddy soil are very few.

Thus, it is both environmentally and industrially important to obtain useful microorganisms in natural environments. Because of the difficulty in isolating and culturing useful microorganisms, however, the fact of the matter is that most of such useful microorganisms has not yet been utilized.

For instance, traditionally, the dilution plating method has been most widely used in order to obtain useful microorganisms from natural environments. (Refer to Nonpatent Reference 1.) This method involves dispersing microorganisms occurring in a natural environment into sterile water and the like to disassemble them into cells as discrete as possible and then plating them uniformly over an agar plate for growing colonies. Subsequently, a number of cells are proliferated from a single cell to form usable colonies.
Nonpatent Reference 1: Koch, R. (1882). Die Aetiologie der Tuberculose, Berl Klin Wochenschr 19, 221-230.

### DISCLOSURE OF THE INVENTION

### PROBLEMS TO BE SOLVED BY THE INVENTION

In the dilution plating method, however, microorganisms are not actually separated into single cells in the process of dispersion, often forming agglomerates each consisting of several to several hundreds of cells, with a result that each colony contains a few tens or more of species of cells in mixture. As the colony proliferates, less competitive microorganisms in it will gradually die out and more competitive ones will only survive. As a result, even if a number of colonies appear in a certain culture medium, many of them will tend to be of the same species and the number of species of microorganisms obtained will be very limited. In other words, the method is useful but has a disadvantage that less competitive microorganisms can hardly be obtained.

As such, it is an object of the present invention to provide a means for conveniently and efficiently isolating and obtaining unknown but useful microorganisms occurring in a natural environment that are less competitive.

### MEANS FOR SOLVING THE PROBLEMS

As a result of intense researches in order to solve the above problem, the present inventors have developed a method for isolation (method for cell enlargement) as a novel method for cell isolation in which culture conditions are combined with cell enlargers, to successfully accomplish the present inventions (1) to (5) below.

The present invention (1) is a method for isolating a desired cell, which comprises selectively applying culture conditions including a culture medium to a sample potentially containing various cells, with addition of a cell enlarger simultaneously with, before or after the application, followed by culturing.

The present invention (2) is the method according to the invention (1) wherein the culture conditions are selectively applied in a various and/or repetitive manner.

The present invention (3) is the method according to the invention (2) wherein the cell enlarger is added in a various and/or repetitive manner.

The present invention (4) is the method according to any one of the inventions (1) to (3) which comprises a step of separating multiple cells linked or coagulated with each other to separate a single cell.

The present invention (5) is the method according to any one of the inventions (1) to (4) which comprises a step of observing the cells after culture under a microscope to suck the single cell with a capillary having a tip inner diameter of 10 to 100 µm or to hold the single cell with another holding device.

Now, definition of each term used in CLAIMS and DESCRIPTION will be described. A "cell" is a morphological and functional constitutional unit for composing a living organism and includes any of eukaryotic cells, prokaryotic cells and archeabacteria. It is also a concept that encompasses any of microorganisms, animal cells and plant cells. A "sample" is not limited as long as it may contain various cells, examples of which may include soil, sand, bottom sludge, tissue and interstitial substance of animals, plant tissues, airborne dust, river water, sea water, food, cosmetics and feedstuff. "Culture conditions" are not particularly limited as long as they are those related to operations or procedures for regulating external conditions for cells to artificially activate, grow and proliferate them, examples of which may include nutritional conditions, pH conditions, temperature conditions and light conditions of culture media and the like. "Selective application" means applying culture conditions as appropriately selected in consideration of cell desired to be obtained. A "cell enlarger" is not particularly limited as long as it is an agent capable of enlarging cells without allowing them to divide, examples of which may include cell division inhibitors such as antibiotics. The term "in a various and/or repetitive manner" in relation to culture conditions means (1) applying multiple sets of culture conditions once, (2) applying one set of culture conditions multiple times and (3) applying multiple sets of culture conditions multiple times, including applying one set of culture conditions multiple times for one culture (each time with a different set of conditions). The term "in a various and/or repetitive manner" in relation to cell enlargers means (1) using multiple cell enlargers once, (2) using one cell enlarger multiple times and (3) using multiple cell enlargers multiple times, including applying one cell enlarger multiple times for one culture (each time with a different cell enlarger). "Multiple cells linked with each other" mean a group of cells whose walls or membranes are linked or attached to each other and "multiple cells coagulated with each other" mean a flora of cells entwined in a complex manner. The term "compatible with a culture medium" means that cells may proliferate, actively absorbing nutrients from the culture medium.

### EFFECT OF THE INVENTION

According to the present invention, various cells (for example, microorganisms) are cultured in a predetermined medium in the presence of cell enlargers, so that cells compatible with the predetermined culture conditions may proliferate and cells compatible with the cell enlargers may grow in size without undergoing cell division. On the contrary, cells not compatible with the predetermined culture conditions may not proliferate and cells not compatible with the cell enlargers may remain relatively small. As such, it will be possible to easily and efficiently obtain cells compatible with the culture conditions and the cell enlargers, for example, by observing under a microscope after culture and obtaining microorganisms larger in size. Further, according to the present invention, it is possible to easily and efficiently obtain cells of a single species (for example, microorganisms) in a reliable manner by combination of predetermined culture conditions and cell enlargers, in comparison with those obtained according to a conventional method such as the dilution plating method. Therefore, when a cell (for example, a microorganism) obtained according to the present invention is cultured, because it is of a completely single species, no competition occurs between species of cells (for example, microorganisms) so that cells of a less competitive species (for example, microorganisms) may grow well. Thus, such an effect may be provided that when novel cells having desired properties are to be obtained, such novel cells (for example, useful microorganisms) having such desired properties may much more likely be found by appropriately selecting culture conditions and cell enlargers reflecting the properties.

### BEST MODE FOR CARRYING OUT THE INVENTION

Now, by way of example of microorganisms as cells, the best mode of the present invention will be described in detail. It is needless to say that the technical scope of the present invention will not be limited to the best mode and that application of the method for isolation according to the present invention to other cells than microorganisms (for example, animal cells and plant cells) may also belong to the technical scope of the present invention.

The present method is a method for isolating desired microorganisms, which comprises selectively applying a culture medium and other culture conditions to a sample potentially containing various microorganisms, with addition of a cell enlarger simultaneously with, before or after the application, followed by culturing. In other words, the present method consists in that desired microorganisms may easily and efficiently be obtained in a reliable manner by determining cell enlargers and culture conditions in relation to the microorganism desired to be obtained.

To describe the present method in detail, first, a culture medium in which microorganisms having desired properties may easily grow is prepared. Next, a sample in which useful microorganisms may occur (for example, soil) is obtained and a small amount of the sample is placed in the culture medium along with a cell enlarger which exhibits a microorganism-enlarging effect (for example, a cell division inhibitor, such as antibiotics for inhibiting cell division) to start cultivation.
After approximately a week, microorganisms viable in this culture medium will grow in size due to the cell division inhibitor. On the other hand, cells of microorganisms nonviable in this culture medium will remain small. Under a microscope, as few cells (one cell) as possible of only the large microbial cells may be taken by micromanipulation into a test tube for cultivation and proliferation to obtain a microbial strain having desired properties.

Here, cell enlargers are not particularly limited as long as they are capable of enlarging cells in relation to microorganisms desired to be obtained, examples of which may include cell division inhibitors, such as antibiotics for inhibiting cell division. Also, the cell enlargers act as bactericides for the microorganisms when their concentrations are high and as cell enlargers for impairing the division function of the microorganisms when the concentrations become lower (the latter being "cell enlargers" as referred to in the present invention). When a cell enlarger is used, cells grow about three to four times larger than normal in length or size. When the cells are placed in another environment where no cell enlargers are present, then they exert cellular functions as usual in the absence of influence by a cell enlarger (reversible). Specifically, known compounds (such as antibiotics and agricultural chemicals) are usable, examples of which include nalidixic acid, pipemidic acid, piromidic acid, Novobiocin, Moxifloxacin, Ciprofloxacin, Benomyl, thiophanate methyl, Thiabendazole, Fuberidazole, Carbendazole, Griseofulvin and Pencycuron. For instance, nalidixic acid is effective against gram-negative bacteria while pipemidic acid, piromidic acid and Novobiocin are effective against gram-positive bacteria. Also, Mixifloxacin is effective against bacilli. Also, multiple cell enlargers may be used in combination when they are known to have common properties against multiple bacterial species. For instance, in EXAMPLE, for the purpose of obtaining bacteria having denitrification ability (found in both gram-positive and gram-negative bacteria) three antibiotics are used in combination to cover both of these bacteria.

Next, it is important to select a culture medium that is compatible with microorganisms desired to be obtained. For instance, when bacteria that carry out photosynthesis and bacteria that do not carry out photosynthesis are to be obtained, a culture medium without an energy source such as glucose can only grow bacteria that carry out photosynthesis while a culture medium containing an energy source such as glucose can only grow bacteria that do not carry out photosynthesis. Those skilled in the art can easily determine which culture medium should be selected when whatever microorganisms are desired to be obtained. For reference, microorganisms desired to be obtained and culture media to be used are exemplified in Table 1.

**Table 1**

| Microorganisms desired to be obtained | Culture media used |
|---|---|
| *Candia* genus | Biggy agar medium |
| *Cryptococcus* genus | Bird seed agar medium |
| *Neisseria* genus | New York City agar medium |
| *Campylobacter* genus | Skirrow's medium |
| *Vibrio* genus | TCBS agar medium |
| *Legionella* genus | BCYE agar medium |
| *Yersinia* genus | CIN agar medium |
| *Corynebacterium* genus, *Lactobacillus* genus, *Micrococcus* genus | CLED agar medium |
| *Haemophilus* genus | Chocolate II agar medium with bacitracin |
| *Pseudomonas aeruginosa* | PASA medium |
| actinomycetes in general | Yeast malt extract agar medium |
| *Streptomyces* genus | albumin medium |

Here, after culturing under the presence of cell enlargers and before separating and obtaining a single enlarged microorganism, the microorganisms after culture may be subjected to a prefractionation process. By this procedure, it may be possible to greatly increase the efficiency in separating and obtaining a single enlarged microorganism. Also, in some cases, the prefractionation process by itself may enable to separate and obtain a single microorganism. Here, prefractionation processes are not particularly limited, examples of which may include fractionation process based on size such as filtering, fractionation process based on weight such as centrifugal separation and flow cytometry.

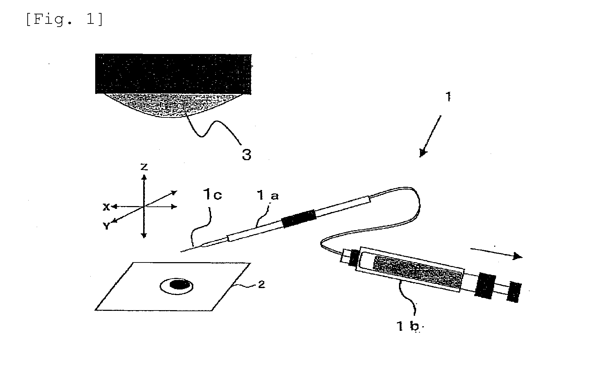

In separating and obtaining an enlarged single microorganism as a result of the culture, it is then preferred to use a holding device capable of holding a single microorganism through physical force such as grasping or suction. Preferred examples of such holding devices may include a micromanipulator capable of sucking a single microorganism with a capillary. Here, such a capillary is preferably made of glass, steel or the like and has an inner diameter of 10 to 100 µm and preferably of 30 to 80 µm. Here, an "inner diameter" means the inner diameter at the tip which may be observed under a microscope. Here, Fig. 1 shows a micromanipulator 1 being used to hold an enlarged single microorganism. As shown, the capillary holder 1a of the micromanipulator 1 is not secured to the body (not shown) and configured to be movable in microns by oil pressure along the XYZ directions. Also, the capillary holder 1a is coupled to an injection cylinder 1b. By manipulating the injection cylinder 1b, the inside of the capillary holder 1a will be held at reduced or increased pressure so that microorganisms may be sucked and released into the holder. To describe a specific procedure for obtaining microorganisms, as shown, a sample after culture is placed on a slide glass 2 and then any enlarged microorganisms in the sample are looked for observing under a microscope 3. In so doing, compared with a traditional method for obtaining single microorganisms in which only living microorganisms are stained, it is easier to find a microorganism since the microorganism itself is larger. After finding such an enlarged microorganism, the capillary holder 1a is appropriately manipulated along the XYZ directions while observing under the microscope so that the capillary 1c at the tip of the capillary holder 1a may contact the enlarged microorganism and the injection cylinder 1b is manipulated along the direction of the arrow in the drawing so that the microorganism may be sucked into the capillary 1c.

As described above, since the microorganism itself has been enlarged, its finding is easy and will be even easier if a dye for staining only living microorganisms is used in combination. Such dyes are not particularly limited as long as they can stain only living microorganisms. Ordinary dyes and fluorescent dyes are usable. Here, fluorescent dyes are particularly preferably used for obtaining microorganisms in a solid such as soil. Here, preferred examples of dyes may include an esterase substrate fluorescent dye CFDA-AM (5-carboxyfluorescein diacetate, acetoxymethyl ester). Other usable fluorescent dyes are listed in Table 2 (excerpted from Microbes and Environments vol. 12, No. 2, 41-56, 1997).

**Table 2**

| fluorescent dye | excitation (nm) | emission (nm) | reference |
|---|---|---|---|
| Fluorescein-isothiocyanate (FITC) | 490 | 520 | protein |
| Lucifer Yellow CH | 435 | 530 | protein |
| Rhodamine 123 | 500 | 540 | mitochondria |
| Acridine Orange | 490 | 530, 640 | nucleic acid |
| Pyronin Y | 540 | 570 | nucleic acid |
| 7-Amino Actinomycin D | 555 | 655 | G-C base pair |
| Chromomycin A3 | 450 | 570 | G-C base pair |
| Mithramycin | 395 | 570 | G-C base pair |
| Olivomycin | 430 | 545 | G-C base pair |
| 4',6-diamidino-2-phenylindole (DAPI) | 372 | 456 | A-T base pair |
| Hoechst 33258 | 365 | 465 | A-T base pair |
| Hoechst 33342 | 355 | 465 | A-T base pair |
| Ethidium bromide | 545 | 605 | double strand nucleic acid |
| Propidium iodide | 530 | 615 | double strand nucleic acid |
| Ethidium homodimer | 528 | 617 | double strand nucleic acid |
| BOBO-1 | 462 | 481 | DNA |
| POPO-1 | 434 | 456 | DNA |
| TOTO-1 | 514 | 533 | DNA |
| YOYO-1 | 491 | 509 | DNA |
| Fluorescein diacetate (FDA) | 495 | 520 | esterase |
| Carboxyfluorescein diacetate (CFDA) | 495 | 520 | esterase |
| Carboxyfluorescein diacetate-acetoxymethylester (CFDA-AM) | 495 | 520 | esterase |
| 5-cyano-2,3-ditolyl tetrazolium chloride (GTC) | 488 | 602 | respiration |
| Tetramethylrhodamine isothiocyanate (TRITC) | 542 | 572 | gene probe |
| Sulforhodamine 101 acid chloride (Texas Red) | 568 | 610 | gene probe |
| Cy3 | 550 | 565 | gene probe |
| 2-hydroxy-3-naphtoic acid-2'-phenylanilide phosphate (HNPP) | 350, 550 | 562 | gene probe |

Also, in obtaining microorganisms in a liquid, ordinary dyes can be used instead of fluorescent dyes. Dyestuffs used for staining (dyes, stains) are not particularly limited, well known examples of which may include Bismarck Brown, carmine, Coomassie Blue, Crystal Violet, eosin, fuchsin, hematoxylin, iodine, Malachite Green, Methyl Green, Methylene Blue, Neutral Red, Nile Blue, Nile Red, rhodamine, safranin, Alizarin Red S and Alcian Blue. These dyestuffs respectively react with and concentrate in various portions of cells and tissues and the differences in their properties assist in revealing particular portions.

In addition, depending on the sample harvested, microorganisms, if untreated, may be coupled or coagulated with each other. (For a soil sample, empirically approximately 90% are in such a condition.) In such a case, a single microorganism must be separated from the microorganism flora. For such separation, it was found that use of a knife and/or needle is efficient.

After obtaining a single microorganism using a micromanipulator, the microorganism is seeded on a predetermined culture medium and cultured for proliferation for a predetermined period of time (for example, one to seven weeks). Thus, since the microorganism is completely of a single species, no competition among species of cells (for example, microorganisms) will occur so that less competitive species of cells (for example, microorganisms) may be proliferated.

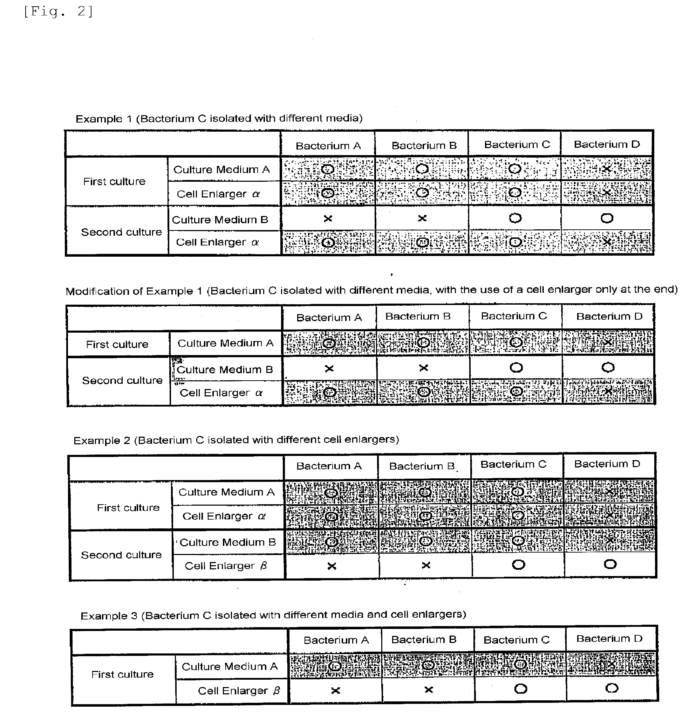

Next, referring to Fig. 2, examples of patterns for obtaining desired microorganisms according to the present method will be described. For the ease of understanding, description will be made on simple models in which four species of microorganisms, namely "Bacterium A" to "Bacterium D" are present in a sample, two culture media, namely, "Culture Medium A" and "Culture Medium B" are used and two cell enlargers, namely, "Cell Enlarger α" and "Cell Enlarger β" are used. Also, in the figure, "○" for a culture medium means that the culture medium is suitable for growth of a bacterium of interest while "×" for a culture medium means that the culture medium is unsuitable for growth of a bacterium of interest. Also, "○" for a cell enlarger means that the cell enlarger is an agent exhibiting a cell-enlarging effect for a bacterium of interest at a concentration of use while "×" for a cell enlarger means that the cell enlarger is an agent toxic for a bacterium of interest (a bactericide) at a concentration of use (in this case, the bacterium dies out or is inactivated) or ineffective against the bacterium of interest. (In this case, the bacterium undergoes cell division as usual.)

First, Example 1 in Fig. 2 shows an example in which Bacterium C is finally isolated by sequentially applying multiple types of media while the type of used cell enlargers remains unchanged. Here, to begin with, in the first culture, Selection Medium A for Bacteria A to C is used under the presence of Cell Enlarger α exhibiting a cell-enlarging effect upon Bacteria A to C. In this case, Bacteria A to C will proliferate and grow enlarger on the nutrition of the culture medium. On the other hand, Bacterium D will die out or be inactivated instead of proliferating because the culture medium is incompatible, with a lack of nutrition. In particular, when Cell Enlarger α acts as a toxin, Bacterium D will die out. Thus, in the first culture, Bacteria A to D will be selected. Next, in the second culture, Selection Medium B for Bacteria C and D is used under the presence of Cell Enlarger α exhibiting a cell-enlarging effect upon Bacteria A to C. In this case, Bacteria C and D may proliferate on the nutrition of the culture medium and, among them, Bacterium C will grow larger because it is compatible with Cell Enlarger α while Bacterium D will proliferate but remain relatively small because it is not compatible with Cell Enlarger α. (In addition, Bacterium D was subjected to an unsuitable medium in the first culture and, therefore, even if bacteria living through dormancy or the like are present, such bacteria have been reduced or inactivated.) Thus, in the second culture, Bacterium C is selected so that only Bacterium C can be isolated.

Here, in Example 1, in order to reliably obtain Bacterium C, cell enlargers are used at each culture step. However, as long as cell enlargers are used at the final step (the second culture for Example 1), theoretically, cell enlargers may not be used at part or all of the culture steps along the way. For instance, as a modification of Example 1, an example is shown in which Cell Enlarger α is not used at the first culture step of Example 1. Thus, theoretically, at the first culture step, Bacteria A to D will be selected by Culture Medium A while Bacterium D will be dormant or die out. Also, cell enlargers are not applied to these bacteria. At the second culture step, Bacteria C and D can grow in Culture Medium B and, since Bacterium D has already died out, only Bacterium C will be selected so that finally Bacterium C will only grow larger by Cell Enlarger α.

Next, Example 2 is an example in which Bacterium C is finally isolated by sequentially applying multiple types of cell enlargers while used media remain unchanged. Here, to begin with, in the first culture, Cell Enlarger α exhibiting a cell-enlarging effect upon Bacteria A to C is used under the presence of Culture Medium A compatible with Bacteria A to C. In this case, Bacteria A to C will grow enlarger on the nutrition of the culture medium. On the other hand, Bacterium D will die out or be inactivated instead of growing larger because the culture medium is incompatible, with a lack of nutrition. In particular, when Cell Enlarger α acts as a toxin, Bacterium D will die out. Next, in the second culture, Cell Enlarger β exhibiting a cell-enlarging effect upon Bacteria C and D is used under the presence of Culture Medium B compatible with Bacteria A to C. In this case, Bacteria A to C may proliferate on the nutrition of the culture medium and, among them, Bacteria A and B will (1) die out or be inactivated when Cell Enlarger β acts as a toxin, or (2) undergo cell division instead of growing larger (in other words, remain relatively small) when Cell Enlarger β does not act as a toxin. Therefore, only remaining Bacterium C can grow even larger in the culture medium so that only Bacterium C can be isolated.

Next, Example 3 is an example in which media and cell enlargers used are narrowed down to isolate Bacterium C through fewer operations (one in this example). Here, Selection Medium A for Bacteria A to C is used as a culture medium. On the other hand, Cell Enlarger β exhibiting enlarging effect upon Bacteria C and D is used as a cell enlarger. Thus, first, when Selection Medium A is used, Bacteria A to C can proliferate on the nutrition of the culture medium. On the other hand, Bacterium D will die out or be inactivated instead of proliferating because the culture medium is incompatible, with a lack of nutrition. Further, when Cell Enlarger β is used, Bacteria C and D can grow larger and, since Bacterium D has died out or been inactivated as described above because the culture medium is incompatible, with a lack of nutrition, only Bacterium C will grow larger on the culture medium. As a result, only Bacterium C can be isolated.

Next, an application example with the use of the present method will be described. First, the present method is useful in bioremediation. Bioremediation is a technique intended for clarification and restoration of environmental contamination of soil, groundwater and the like by decomposing and detoxifying contaminants by utilizing actions of microorganisms and the like. Discovery of microorganisms involved in the decomposition and detoxification of such contaminants may efficiently be carried out. Further, these methods are also useful in discovery of microorganisms essential for creating new drugs. In particular, actinomycetes are known to produce a number of useful and important substances essential for pharmaceuticals. It is, therefore, important to search for new species of actinomycetes. As such, soil or the like in which actinomycetes are contained is placed in a culture medium which allows only the actinomycetes to grow, with addition of cell division inhibitors, so that only the actinomycetes may grow larger. Single cells of the actinomycetes are obtained and cultured according to the present method so that various actinomycetes, including unknown ones, may efficiently be obtained.

### EXAMPLES

It is believed that denitrificans in rice paddy soil suppress contamination of groundwater with nitrate nitrogen due to excessive fertilization and/or suppress generation of N₂O nitrogen oxide having a very strong greenhouse effect. Although a large number of bacteria exhibiting a denitrification effect in laboratory are known to exist, number of bacteria known to exhibit a denitrification effect in rice paddy soil has been limited. This is due to that only competitive bacteria can be grown by the conventional methods and that bacteria showing a denitrification effect in rice paddy soil cannot be identified.
Using the method as described in [0033], the present inventors found that a strain of bacteria from rice paddy soil showed denitrification effect, though the strain had not been believed to have the effect. In the future, more new bacteria possessing a denitrification effect (denitrificans) will be discovered from rice paddy soil using the present technique.

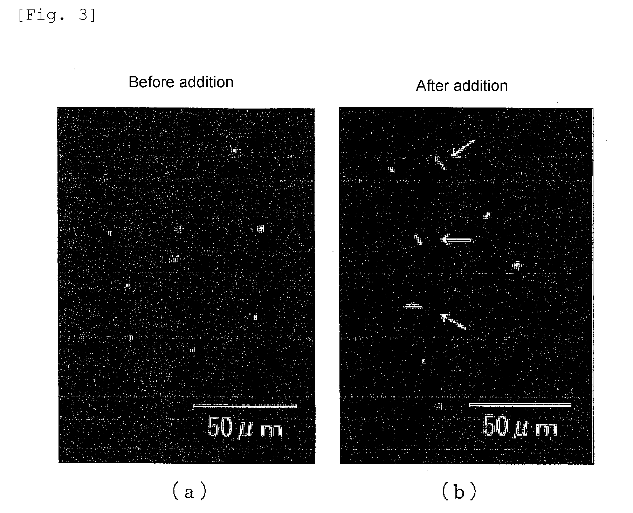

As a laboratory model of rice paddy soil where denitrification is active, a 10 mL vial bottle was filled with slightly less than 10 mL of soil (rice paddy soil from Tanashi Farm of the University of Tokyo), a selective substrate of denitrificans (sodium succinate, approximately 5 g/L) and sodium nitrate (approximately 5 g/L) and the gas phase was completely substituted with argon-acetylene. Culture was then carried out anaerobically at 30°C. After culturing for 24 hours, the substrate (sodium succinate) and sodium nitrate were again added so that the final concentration of each was approximately 5 g/L and simultaneously three cell enlargers, namely nalidixic acid (80 µg/g of soil), pipemidic acid (40 µg/g of soil) and piromidic acid (40 µg/g of soil) were added, followed by further culturing anaerobically at 30°C for 24 hours. A fluorescent dye CFDA-AM(final concentration 50 µM) was added to the cultured product and the product was observed under a fluorescent microscope (excited in blue, irradiated with blue light) (Fig. 3(b)). In combination, as a comparative example, the fluorescent dye was added and observation was carried out under a microscope with no cell enlargers added (Fig. 3(a)). As a result, in Fig. 3(a) only round or oval microbial cells were observed in the sample, while in Fig. 3(b) elongated and larger cells were also observable (indicated by arrows).
From the sample to which the cell enlargers and the fluorescent dye had been added, 130 strains (a "strain" is a population of genetically homogenous microorganisms) of enlarged cells were separated by a micromanipulator under a fluorescent microscope. When the strains were cultured in an LB liquid medium for one week, 82 strains grew and proliferated. The strains that showed proliferation were cultured in a Giltay liquid medium for one week and assayed for the presence or absence of a denitrification effect on the basis of gas generation and discoloration of the culture medium. 50 strains were then determined to possess a denitrification effect. Eight among the 50 strains that were observed with the denitrification effect were proliferated and phylogenetically analyzed on the basis of 16S rDNA base sequence of the bacteria.

As a result of the phylogenetic analysis, it was determined that they consist of five bacteria as follow: *Stenotrophomonas sp.* (two strains), *Ochrobactrum anthropi* (one strain), *Burkholderia cepacia.* (two strains), *Pseudomonas putida.* (two strains) and *Pseudomonas sp.* (one strain). The first two of them, that is, *Stenotrophomonas* sp. and *Ochrobactrum anthropi.* have not traditionally been supposed to possess a denitrification effect, but have been found anew to possess a denitrification effect. The three others including *Burkholderia cepacia.* have traditionally been recognized with a denitrification effect as strains cultured in laboratories, and have easily been confirmed with their denitrification effects on a specific sample from rice paddy soil by application of the method according to the present invention.

By way of example of EXAMPLE described above, the present technique can provide a means extremely useful in isolation, culture and utilization of useful microorganisms, which is applicable in a wide variety of fields such as bioremediation of soil, biodesulfurization of petroleum and manufacture of pharmaceuticals, using microorganisms.

### BRIEF DESCRIPTION OF THE DRAWINGS

Fig. 1 is an illustration showing a micromanipulator being used to obtain an enlarged single microorganism.
Fig. 2 shows examples of patterns for obtaining desired microorganisms according to the present method. Here, in order to obtain Bacterium C, three examples are shown in which media and cell enlargers are used in different combinations.
Fig. 3 shows microphotographs showing the effect of cell enlargers upon microorganisms in soil (Fig. 3(a) before addition, Fig. 3(b) after addition).

### DESIGNATION OF REFERENCE NUMERALS

1: micromanipulator
1a: capillary holder
1b: injection cylinder
1c: capillary
2: slide glass
3: objective of microscope

## Claims

1. A method for isolating a desired cell, which comprises selectively applying culture conditions including a culture medium to a sample potentially containing various cells, with addition of a cell enlarger simultaneously with, before or after the application, followed by culturing.

2. The method according to Claim 1, wherein the culture conditions are selectively applied in a various and/or repetitive manner.

3. The method according to Claim 2, wherein the cell enlarger is added in a various and/or repetitive manner.

4. The method according to any one of Claims 1 to 3, which comprises a step of separating multiple cells linked or coagulated with each other to separate single cell.

5. The method according to any one of Claims 1 to 4, which comprises a step of observing the cells after culture under a microscope to suck the single cell with a capillary having a tip inner diameter of 10 to 100 µm or to hold the single cell with the other holding devices.
